# EUROPEAN PATENT SPECIFICATION

(11) **EP 1 494 658 B1**
(45) Date of publication and mention of the grant of the patent: **04.01.2006**
(21) Application number: 02730093.8
(22) Date of filing: 03.04.2002
(51) Int. Cl.: A61K 31/05, A61K 31/222, A61K 31/23, A61K 31/231, A61K 31/232, A23L 1/30, A61P 25/28, A61P 25/16, A61P 9/10

(54) **NATURAL PHENOLIC PRODUCTS AND DERIVATIVES THEREOF FOR PROTECTION AGAINST NEURODEGENERATIVE DISEASES**
NATÜRLICHE PHENOLISCHE PRODUKTE UND DEREN DERIVATE ZUM SCHUTZ VOR NEURODEGENERATIVEN ERKRANKUNGEN
PRODUITS PHENOLIQUES NATURELS ET LEURS DERIVES PERMETTANT D'OBTENIR UNE PROTECTION CONTRE LES MALADIES NEURODEGENERATIVES

(43) Date of publication of application: 12.01.2005
(73) Proprietor: PULEVA BIOTECH, S.A., E-18004 Granada (ES)
(72) Inventor: GEERLINGS, Arjan, E-18006 GRANADA (ES); LOPEZ-HUERTAS LEON, Eduardo, E-18200 MARACENA (Granada) (ES); MORALES SANCHEZ, Juan-Carlos, E-18194 CULLAR-VEGA (Granada) (ES); BOZA PUERTA, Julio, 18008 Granada (ES); JIMENEZ LOPEZ, Jesus, E-18320 SANTA FE (Granada) (ES)
(74) Representative: Portela Gasch, Sergio Raul
(86) International application number: PCT/EP2002/003675
(87) International publication number: WO 2003/082259

(56) References cited:
- EP-A- 1 072 265
- WO-A-02/18310
- WO-A-99/62336
- WO-A1-03/082798
- GB-A- 2 345 058
- US-A- 5 114 716
- PATENT ABSTRACTS OF JAPAN vol. 1995, no. 11, 26 December 1995 (1995-12-26) & JP 07 223940 A (POLA CHEM IND INC), 22 August 1995 (1995-08-22)
- ARUOMA, OKEZIE I.: "Antioxidants and functional food science: a case for ergothioneine and hydroxytyrosol" FREE RADICALS IN CHEMISTRY, BIOLOGY AND MEDICINE, 2000, pages 439-452, XP008010844
- DATABASE WPI Week 199527, 9 May 1995 (1995-05-09) Derwent Publications Ltd., London, GB; AN 1995-203735 XP002222976 NISHIMURA KEIICHI; KITADA YOSHIO ET AL.: "Thrombosis suppressor and composition containg the same" & JP 07 118146 A (POLA CHEM IND INC), 9 May 1995 (1995-05-09)
- MANNA C ET AL: "Biological effects of hydroxytyrosol, a polyphenol from olive oil endowed with antioxidant activity." ADVANCES IN EXPERIMENTAL MEDICINE AND BIOLOGY. UNITED STATES 1999, vol. 472, 1999, pages 115-130, XP008010843 ISSN: 0065-2598
- OWEN R W ET AL: "Olive-oil consumption and health: the possible role of antioxidants." THE LANCET ONCOLOGY. ENGLAND OCT 2000, vol. 1, October 2000 (2000-10), pages 107-112, XP001128126 ISSN: 1470-2045
- PATENT ABSTRACTS OF JAPAN vol. 2002, no. 09, 4 September 2002 (2002-09-04) & JP 2002 153238 A (NIPPON SHINYAKU CO LTD), 28 May 2002 (2002-05-28)
- HASHIMOTO TSUNEICHI ET AL: "Protective role of endogenous polyphenol 3,4-dihydroxyphenylethanol against hydrogen peroxide-induced cell damage in PC12 cells." JAPANESE JOURNAL OF PHARMACOLOGY, vol. 88, no. Supplement 1, 2002, page 140P XP001128709 75th Annual Meeting of the Japanese Pharmacological Society;Kumamoto, Japan; March 13-15, 2002, 2002 ISSN: 0021-5198
- ACEVEDO L ET AL: "New phenylethanoids from Buddleja cordata subsp. cordata." PLANTA MEDICA, (2000 APR) 66 (3) 257-61. , XP001128125
- TSUJIMOTO, TAKASHI ET AL: "Crosslinkable polyphenols from urushiol analogues" MACROMOLECULAR CHEMISTRY AND PHYSICS (2001), 202(17), 3420-3425 , XP001126939
- DATABASE WPI Week 198333, Derwent Publications Ltd., London, GB; Class C03, AN 1983-738596 & JP 58 116 637 A (NICHIHAISHA EBI SHI; NIPPON SAIBAI SUISAN KK) 11 July 1983

## Description

### FIELD OF THE INVENTION

The present invention relates to the use of phenolic derivatives for protection against neurodegenerative diseases and to compositions comprising these compounds.

### BACKGROUND OF THE INVENTION

During life, cells in all biological systems are exposed to oxidation. Oxidation leads to the formation of free radicals, which are compounds that contain one, or more unpaired electrons. These radicals react easily with other molecules in the cell, and during life the continuous attack of free radicals may alter cellular mechanisms and ultimately may lead to cell death. Free radicals and molecules that generate them are often classified as reactive oxygen species (ROS) and cells have developed mechanisms to cope with these compounds. However, problems may arise when these mechanisms are not sufficient to eliminate all ROS present. Under these conditions oxidative stress may cause cellular damage and subsequent cellular death, mainly by apoptosis. One of the principle factors that reduce ROS and oxidative stress are antioxidants. Cells produce their own antioxidants (e.g. glutathione) but may also acquire dietary antioxidants.

The brain is particularly vulnerable to oxidative stress for several reasons. First of all, because of its high-energy demand, the brain consumes 20% of all oxygen used. The consumption of oxygen leads to the production of ROS and therefore the brain generates high amounts of these reactive oxygen species. Additionally, excitatory amino acids (e.g. glutamate) generate high levels of ROS on their massive release after brain injury. Glutathione is the main antioxidant in brain. After 30 years of age, the concentration of this antioxidant in human brain declines. Therefore, it is believed that during aging oxidative stress becomes more abundant. In addition, due to the blood brain barrier, many dietary antioxidants (like vitamin C, carotenoids and flavonoids) are unable to enter the brain.
Certain diseases of the brain and nervous system are thought to involve free radical processes and oxidative damage, either as a primary cause or as a consequence of the disease. Examples of brain disease where oxidative stress plays an important role are Alzheimer disease, cognitive dysfunction (loss of memory), and Parkinson disease. For the treatment and prevention of these diseases, antioxidants might be useful. Since most dietary antioxidants cannot pass the blood brain barrier, there is a need for antioxidants that penetrate this biological barrier (Gilgun-Sherki et al. Neuropharmacology 2001, 40(8), 959-75). The use of antioxidants, however, is already known in the art in the prevention of cardiovascular diseases (WO 97/0667 and WO 01/45514).

On the other hand, several studies have demonstrated that copper (Cu²⁺), zinc (Zn²⁺) and iron (Fe³⁺) ions play an important role in Alzheimer's and Parkinson's disease. Concentrations of these ions are elevated in beta-amyloid plaques and in Lewy bodies, the beta-amyloid analogue in Parkinson's disease. Moreover, these metal ions may initiate the aggregation process of beta-amyloid peptides leading to insoluble fibril formation. This has led some investigators to study the effect of metal chelators on the formation of beta-amyloid plaques. The results of in vivo studies demonstrated that a zinc and copper chelator reduced amyloid deposits in a mouse model for Alzheimer's disease (Cherny et al. Neuron 2001, 30(3), 665-76). Therefore, metal chelators are thought to have neuroprotective properties.

Basic and clinical researchers have been investigating on neurodegenerative disease, and on how to protect neurons from dying, a principal hallmark of these diseases. Since oxidative stress is a common theme in many neurodegenerative diseases and may lead to neuronal death, some antioxidants have been investigated for their potential to reduce oxidative stress. Cases of partial improvement have been reported, but to date no potent antioxidants have been found that fulfil the basic requirements for their use in the treatment of neurodegenerative disease; being that they need to:
1. enter the brain in sufficient amounts
2. have antioxidant activity once inside the central nervous system
3. be non-toxic

Moreover, it would be advantageous if the antioxidant is a natural product known for its presence in dietary products. Most antioxidants present in food however do not fulfil the first requirement since they are unable to pass the blood brain barrier.

Another important aspect of many neurodegenerative diseases is the accumulation of insoluble protein aggregates (e.g. beta-amyloid plaques in Alzheimer's disease and Lewy bodies in Parkinson's disease). The exact mechanism of how these aggregates are formed is still not completely understood, but metal ions are thought to play an important role. For instance, zinc and copper ions in low concentrations facilitate the formation of beta-amyloid plaques, and iron, among other metal ions, is found in high concentrations in Lewy bodies. Chelators of these metal ions are known to dissolve above-mentioned aggregates and therefore are useful as neuroprotectants in neurodegenerative disease. To be useful in neuroprotection, chelators of metal ions need to fulfil three requirements, they need to:
1. readily enter the brain in sufficient amounts
2. solubilize protein aggregates in vivo
3. be non-toxic

In this invention we describe a natural product isolated from olive (2-(3,4-dihydroxyphenyl) ethanol, hydroxytyrosol (reference compound)) that combines all above-mentioned requirements for its use in a treatment against neurodegenerative disease. This compound readily enters the brain, is a potent antioxidant even when it is localized in the brain, and chelates metal ions. Moreover, hydroxytyrosol is a potent inhibitor of monoamine oxidase (MAO-B). This combination of characteristics makes hydroxytyrosol suitable for the treatment of Alzheimer's, Parkinson's and other neurodegenerative diseases. Moreover, this compound is a natural product and can be isolated from olive. Therefore, this compound may be used as a dietary supplement or in pharmaceutical preparations. We also describe a series of hydroxytyrosol esters that upon their hydrolysis, for instance in the stomach of a consumer, liberates hydroxytyrosol. These esters are very useful for the conservation of hydroxytyrosol, because in the ester form, hydroxytyrosol is prevented from oxidation. Another advantage of using hydroxytyrosol esters is their lipid solubility. Finally, we describe another natural occurring phenolic compound, tyrosol (2-(4-hydroxyphenyl) ethanol) (reference compound), which is a powerful antioxidant and readily enters the nervous system. This compound is also useful in the treatment of neurodegenerative diseases where oxidative stress plays an important role.

Patent application JP -A- 7223940 discloses that hydroxytyrosol is effective in treating various diseases related to active oxygen such as inflammation, senile dementia, ischeamic disease, etc. Publication "Free Radicals is Chemistry, Biology and Medicine" (2000) discloses in an article by O.I. Aruoma in pages 439-452, that hydroxytyrosol, present in olive oil, protects against oxidative DNA damage in cells by scavenging ONOO⁻. Patent application JP-A-7118146 discloses pharmaceuticals or foods for treating brain disorders comprising hydroxytyrosol. Finally, Owen discloses in The Lancet Oncology, 1, 107-112, 2000 that hydroxytyrosol and tyrosol are potent antioxidants which reduce oxidative stress.

WO-A-03082798, to the Applicant, claiming the priority date of 03.04.2002, discloses phenolic derivatives/esters of tyrosol and hydroxytyrosol and their use in pharmaceutical or food compositions for the prevention and treatment of cardiovascular, hepatic and renal disorders, diabetes and acute or chronic inflammatory processes.

### SUMMARY OF THE INVENTION

The present invention provides, therefore, phenolic derivatives for protection against neurodegenerative diseases and compositions comprising these compounds.

An aspect of the invention relates to phenolic derivatives for the treatment or prevention of neurodegenerative diseases such as: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

A second aspect of the invention relates to compositions comprising phenolic derivatives for the treatment or prevention of neurodegenerative diseases.

### DETAILED DESCRIPTION OF THE INVENTION

As mentioned above, the first aspect of the invention relates to phenolic derivatives for the treatment or prevention of neurodegenerative diseases. Said diseases are, for instance, Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

Two phenolic compounds, hydroxytyrosol and tyrosol (reference compounds), are both capable of entering the brain in relatively large quantities and act as antioxidants. Furthermore, hydroxytyrosol acts as an inhibitor of MAO-B and as a chelator of iron, zinc and copper ions. Because of these characteristics both phenolic compounds function as neuroprotectants in the therapy of neurodegenerative diseases.

The object of this invention is to increase the antioxidant levels in brain and therefore to reduce the oxidative stress in this tissue. A second objective of this invention is to reduce the concentrations of iron, zinc and copper ions in brain, and by that means inhibit the formation of protein precipitates. Hydroxytyrosol, tyrosol, and compounds that liberate these phenolics after their hydrolysis can be administered as a dietary supplement or as a pharmaceutical preparation and may, thus, be used in the treatment of diseases where oxidative stress, insoluble protein deposits, and subsequent neuronal loss play an important role.

Phenolic compounds can be found among others in olive, red wine, black tea, and in a wide variety of fruits and vegetables. One of the major phenolic compounds from olive is hydroxytyrosol, which is a very powerful antioxidant. It can be isolated from the leaf of the olive tree but is also found in the olive fruit and in olive oil. The health benefits of the Mediterranean diet are partly due to the consumption of olive oil and the presence of phenolic compounds therein.

Our studies showed that, when taken orally, hydroxytyrosol (reference compound) is rapidly taken up from the intestinal tract and subsequently is able to pass the blood brain barrier in relatively large amounts. For this reason this compound accumulates in the cerebrospinal fluid and from there it has access to the cells in the central nervous system. Interestingly, cerebrospinal fluid taken from rats after oral administration of mentioned compound showed a considerable increase in total antioxidant capacity. This makes hydroxytyrosol very useful in the treatment of neurological diseases where oxidative stress plays an important role.

Different kind of stressors may induce oxidative stress and oxidative damage that leads to cell death. In Alzheimer's disease, the beta-amyloid peptide accumulates in brain tissue. This accumulation may lead to oxidative stress, and large amounts of cells die leading to the typical symptoms associated to Alzheimer's disease. Glutamate is the major excitatory neurotransmitter in brain, but is also a powerful oxidant. Furthermore, different mechanisms may induce the production of hydrogen peroxide which is a very potent prooxidant in cells. We found that hydroxytyrosol protects cells from the central nervous system from dying after exposure to mentioned stressors (beta-amyloid, glutamate and hydrogen peroxide). This, together with the fact that hydroxytyrosol easily enters the brain, makes it a powerful neuroprotectant. Since oxidative stress plays an important role in many neurodegenerative diseases and consequently large amounts of cells die, this phenolic compound is especially useful in the treatment of these diseases.

Besides its antioxidant capacity, we found that hydroxytyrosol is a chelator of iron, zinc and copper ions. Since zinc, copper and iron ions induce the formation of beta-amyloid plaques, chelators of these ions might solubilize these plaques. This is thought to be beneficial for Alzheimer disease therapy (Chemy et al. Neuron 2001, 30(3), 665-76). Hydroxytyrosol not only is a strong chelator of iron, zinc and copper ions, it does indeed solubilize beta-amyloid deposits. Therefore, hydroxytyrosol has a dual neuroprotective action, it is a potent antioxidant and reduces iron, zinc and copper concentrations in certain brain regions. Both actions are beneficial in neurodegenerative disease, and so this phenolic compound is advantageous over compounds that are solely antioxidants or chelators of metal ions.

In Parkinson disease large amounts of dopaminergic neurons die in the substantia nigra. Dopamine replacement therapy, in the form of levodopa supplementation, has been used successfully in the treatment of Parkinson disease. Inhibition of dopamine metabolism is a valuable adjunct to exogenous dopaminergic replacement. Therefore, inhibitors of MAO-B have been used to treat early and advanced Parkinson disease for a number of years. Although some controversy remains, existing evidence raises the possibility that MAO-B inhibition may confer a protective effect in Parkinson disease, delaying the progression of the underlying pathology. We found that hydroxytyrosol is a strong inhibitor of MAO-B. Therefore, this activity of hydroxytyrosol may be of additional use in the treatment of this disease.

A second neuroprotector is tyrosol (reference compound). Tyrosol is also a phenolic compound found in olive. As hydroxytyrosol, tyrosol is a powerful antioxidant and is rapidly taken up from the intestinal tract. Moreover, it is capable of passing the blood brain barrier in relatively large amounts. We detected tyrosol in cerebrospinal fluid of rats after having received tyrosol orally. Therefore, tyrosol is very useful in the treatment of neurodegenerative diseases where oxidative stress plays an important role. Since tyrosol increases the total antioxidant levels of the brain, it can be used to treat disorders like Alzheimer's and Parkinson's disease. It can also be used to treat stroke and loss of memory.

This invention provides hydroxytyrosol and tyrosol esters for the treatment or prevention of neurodegenerative diseases. These esters have several advantages over hydroxytyrosol and tyrosol. They are more lipid soluble and the hydroxyl groups of hydroxytyrosol and tyrosol are protected from oxidation by a fatty acid ester. The ester bond is rapidly hydrolysed in an acid environment liberating the fatty acid and hydroxytyrosol or tyrosol.

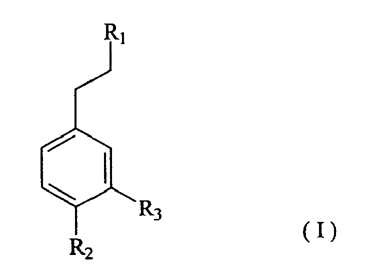

The hydroxytyrosol and tyrosol fatty acid esters described in this invention have a common structure as represented by formula I. Within this structure groups R1, R2, and R3 vary leading to a series of related hydroxytyrosol or tyrosol derivatives. Groups R1 and R2 can be either a hydroxyl group or a hydroxyl group protected with a fatty acid chain via an ester bond. Group R3 can be either a hydrogen (in the case of tyrosol esters), a hydroxyl group (in the case of hydroxytyrosol esters) or a hydroxyl group protected with a fatty acid chain via an ester bond (in the case of hydroxytyrosol esters). Each compound must contain one, two or three fatty acid chains with a length from C2 to C22. Therefore, R₁ and R₂ are independently selected from: OH, OCOalkyl or OCOalkenyl, and R₃ is either H, OH, OCOalkyl or OCOalkenyl, and wherein the alkyl or alkenyl group contains from 2 to 22 carbon atoms.

This invention consequently provides hydroxytyrosol and tyrosol esters for the treatment or prevention of neurodegenerative diseases. These esters have several advantages over hydroxytyrosol and tyrosol. They are more lipid soluble and the hydroxyl groups of hydroxytyrosol and tyrosol are protected from oxidation by a fatty acid ester. The ester bond is rapidly hydrolysed in an acid environment liberating the fatty acid and hydroxytyrosol or tyrosol. These esters are: 2-(3,4-dihydroxyphenyl) ethyl acetate (V), 2-(3,4-dihydroxyphenyl) ethyl oleate (VI), 2-(3,4-dihydroxyphenyl) ethyl stearate (VII), 2-(3,4-dihydroxyphenyl) ethyl docosahexaenoate (VIII), 2-(3,4-dihydroxyphenyl) ethyl eicosapentaenoate (IX), 2-(4-hydroxyphenyl) ethyl acetate (X), 2-(4-hydroxyphenyl) ethyl oleate (XI), 2-(4-hydroxyphenyl) ethyl docosahexaenoate (XIII), 2-(4-hydroxyphenyl) ethyl eicosapentaenoate (XIV), 2-(3,4-diacetoxyphenyl) ethyl acetate (XV), 2-(3,4-dioleyloxyphenyl) ethyl oleate (XVI), 2-(3,4-distearyloxyphenyl) ethyl stearate (XVII), 2-(3,4-didocosahexaenoyloxyphenyl) ethyl docosahexaenoate (XVIII), 2-(3,4-dieicosapentaenoyloxyphenyl) ethyl eicosapentaenoate (XIX), 2-(4-acetoxyphenyl) ethyl acetate (XX), 2-(4-oleyloxyphenyl) ethyl oleate (XXI), 2-(4-stearyloxyphenyl) ethyl stearate (XXII), 2-(4-docosahexaenoyloxyphenyl) ethyl docosahexaenoate (XXIII), and 2-(4-eicosapentaenoyloxyphenyl) ethyl eicosapentaenoate (XXIV)

Whereas compounds (1) (V), (6) (X) and (7) (XI) are natural products, compounds (8) (XII), (11) (XV) and (16) (XX) have not been found in nature. Compounds (1) (V) to (20) (XXIV) have been synthesised following standard esterification methods. The synthesis of some of these compounds, namely the compounds of formulae V, VI, VII, XV and XVII, are exemplified below.

This invention also provides the use of phenolic derivatives, selected from the group consisting of: 2-(3,4-dihydroxyphenyl) ethyl acetate (V), 2-(3,4-dihydroxyphenyl) ethyl oleate (VI), 2-(3,4-dihydroxyphenyl) ethyl stearate (VII), 2-(3,4-dihydroxyphenyl) ethyl docosahexaenoate (VIII), 2-(3,4-dihydroxyphenyl) ethyl eicosapentaenoate (IX), 2-(4-hydroxyphenyl) ethyl acetate (X), 2-(4-hydroxyphenyl) ethyl oleate (XI), 2-(4-hydroxyphenyl) ethyl stearate (XII), 2-(4-hydroxyphenyl) ethyl docosahexaenoate (XIII), 2-(4-hydroxyphenyl) ethyl eicosapentaenoate (XIV), 2-(3,4-diacetoxyphenyl) ethyl acetate (XV), 2-(3,4-dioleyloxyphenyl) ethyl oleate (XVI), 2-(3,4-distearyloxyphenyl) ethyl stearate (XVII), 2-(3,4-didocosahexaenoyloxyphenyl) ethyl docosahexaenoate (XVIII), 2-(3,4-dieicosapentaenoyloxyphenyl) ethyl eicosapentaenoate (XIX), 2-(4-acetoxyphenyl) ethyl acetate (XX), 2-(4-oleyloxyphenyl) ethyl oleate (XXI), 2-(4-stearyloxyphenyl) ethyl stearate (XXII), 2-(4-docosahexaenoyloxyphenyl) ethyl docosahexaenoate (XXIII), and 2-(4-eicosapentaenoyloxyphenyl) ethyl eicosapentaenoate (XXIV), characterised in that they liberate hydroxytyrosol or tyrosol after their hydrolysis, in the preparation of a composition for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

Illustrations of some hydroxytyrosol and tyrosol derivatives are:
(1) 2-(3,4-Dihydroxyphenyl) ethyl acetate, an hydroxytyrosol derivative having the formula V, wherein R2 and R3 are hydroxyl groups, and R1 is an hydroxyl group protected with an acetic acid chain via an ester bond.
(2) 2-(3,4-Dihydroxyphenyl) ethyl oleate, an hydroxytyrosol derivative having the formula VI, wherein R2 and R3 are hydroxyl groups, and R1 is an hydroxyl group protected with an oleic acid chain via an ester bond.
(3) 2-(3,4-Dihydroxyphenyl) ethyl stearate, an hydroxytyrosol derivative having the formula VII, wherein R1 and R2 are hydroxyl groups, and R3 is an hydroxyl group protected with a stearic acid chain via an ester bond.
(4) 2-(3,4-Dihydroxyphenyl) ethyl docosahexaenoate, an hydroxytyrosol derivative having the formula VIII, wherein R1 and R2 are hydroxyl groups, and R3 is an hydroxyl group protected with a docosahexaenoic acid chain via an ester bond.
(5) 2-(3,4-Dihydroxyphenyl) ethyl eicosapentaenoate, an hydroxytyrosol derivative having the formula IX, wherein R1 and R2 are hydroxyl groups, and R3 is an hydroxyl group protected with an eicosapentaenoic acid chain via an ester bond.
(6) 2-(4-Hydroxyphenyl) ethyl acetate, a tyrosol derivative having the formula X, wherein R2 is an hydroxyl group, R3 is an hydrogen, and R1 is an hydroxyl group, protected with an acetic acid chain via an ester bond.
(7) 2-(4-Hydroxyphenyl) ethyl oleate, a tyrosol derivative having the formula XI, wherein R2 is an hydroxyl group, R3 is an hydrogen, and R1 is an hydroxyl group protected with an oleic acid chain via an ester bond.
(8) 2-(4-Hydroxyphenyl) ethyl stearate, a tyrosol derivative having the formula XII, wherein R2 is an hydroxyl group, R3 is an hydrogen, and R1 is an hydroxyl group protected with a stearic acid chain via an ester bond.
(9) 2-(4-Hydroxyphenyl) ethyl docosahexaenoate, a tyrosol derivative having the formula XIII, wherein R2 is an hydroxyl group, R3 is an hydrogen, and R1 is an hydroxyl group protected with a docosahexaenoic acid chain via an ester bond.
(10) 2-(4-Hydroxyphenyl) ethyl eicosapentaenoate, a tyrosol derivative having the formula XIV, wherein R2 is an hydroxyl group, R3 is an hydrogen, and R1 is an hydroxyl group protected with an eicosapentaenoic acid chain via an ester bond.
(11) 2-(3,4-Diacetoxyphenyl) ethyl acetate, an hydroxytyrosol derivative having the formula XV, wherein R1, R2, and R3 are hydroxyl groups protected with an acetic acid chain via an ester bond.
(12) 2-(3,4-Dioleyloxyphenyl) ethyl oleate, an hydroxytyrosol derivative having the formula XVI, wherein R1, R2, and R3 are hydroxyl groups protected with an oleic acid chain via an ester bond.
(13) 2-(3,4-Distearyloxyphenyl) ethyl stearate, an hydroxytyrosol derivative having the formula XVII, wherein R1, R2, and R3 are hydroxyl groups protected with a stearic acid chain via an ester bond.
(14) 2-(3,4-Didocosahexaenoyloxyphenyl) ethyl docosahexaenoate, an hydroxytyrosol derivative having the formula XVIII, wherein R1, R2, and R3 are hydroxyl groups protected with a docosahexaenoic acid chain via an ester bond.
(15) 2-(3,4-Dieicosapentaenoyloxyphenyl) ethyl eicosapentaenoate, an hydroxytyrosol derivative having the formula XIX, wherein R1, R2, and R3 are hydroxyl groups protected with an eicosapentaenoic acid chain via an ester bond.
(16) 2-(4-Acetoxyphenyl) ethyl acetate, a tyrosol derivative having the formula XX, wherein R3 is an hydrogen; and wherein R1 and R2 are hydroxyl groups protected with an acetic acid chain via an ester bond.
(17) 2-(4-Oleyloxyphenyl) ethyl oleate, a tyrosol derivative having the formula XXI, wherein R3 is an hydrogen, and wherein R1 and R2 are hydroxyl groups protected with an oleic acid chain via an ester bond.
(18) 2-(4-Stearyloxyphenyl) ethyl stearate, a tyrosol derivative having the formula XXII, wherein R3 is an hydrogen, and wherein R1 and R2 are hydroxyl groups protected with a stearic acid chain via an ester bond.
(19) 2-(4-Docosahexaenoyloxyphenyl) ethyl docosahexaenoate, a tyrosol derivative having the formula XXIII, wherein R3 is an hydrogen, and wherein R1 and R2 are hydroxyl groups protected with a docosahexaenoic acid chain via an ester bond.
(20) 2-(4-Eicosapentaenoyloxyphenyl) ethyl eicosapentaenoate, a tyrosol derivative having the formula XXIV, wherein R3 is an hydrogen, and wherein R1 and R2 are hydroxyl groups protected with a eicosapentaenoic acid chain via an ester bond.

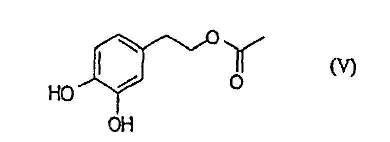

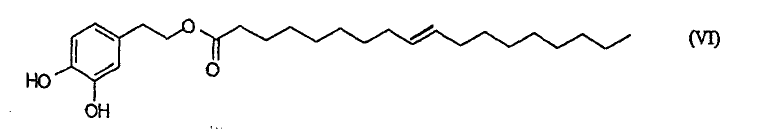

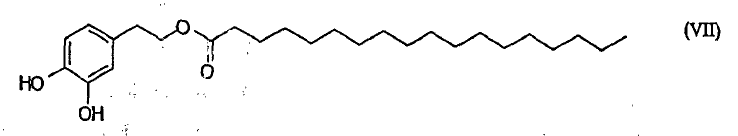

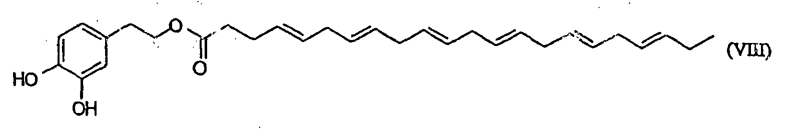

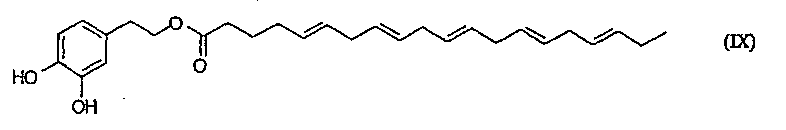

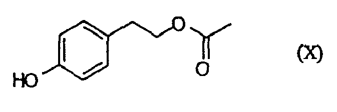

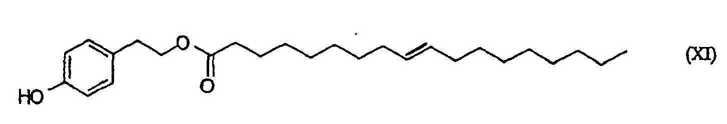

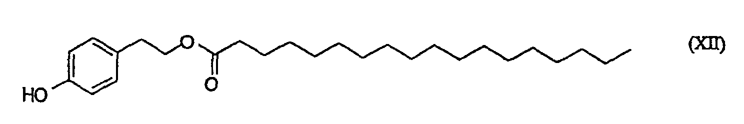

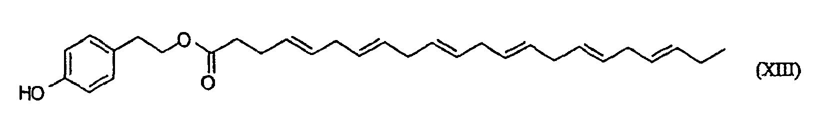

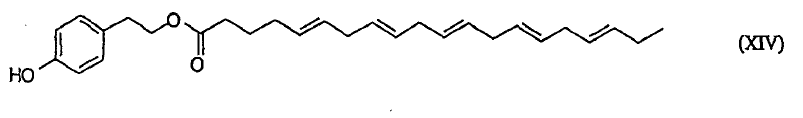

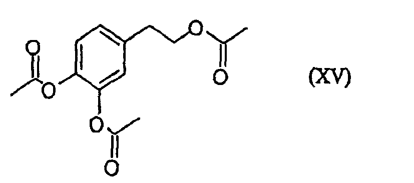

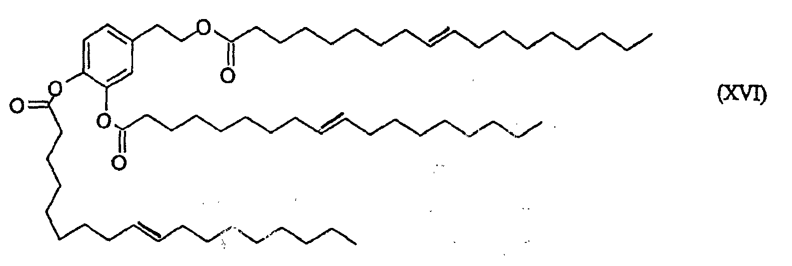

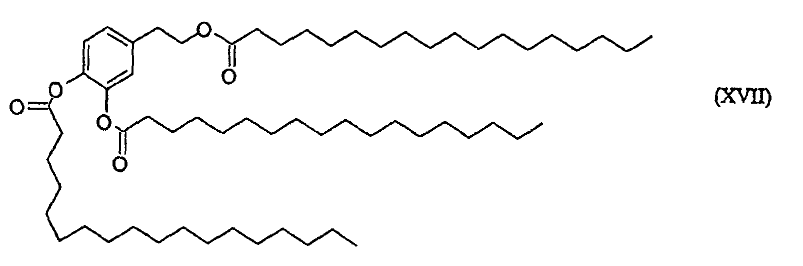

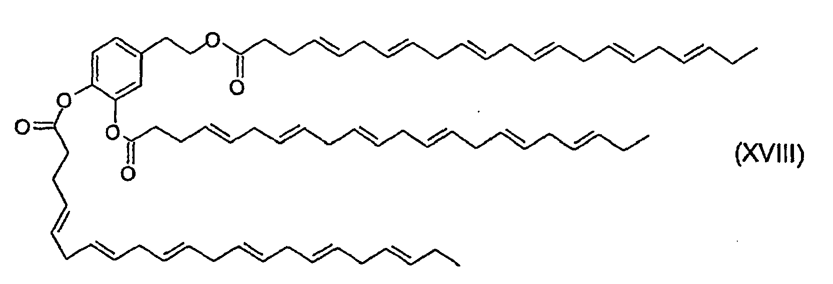

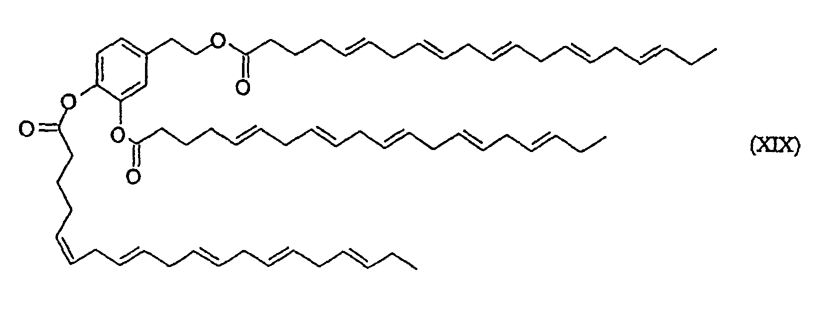

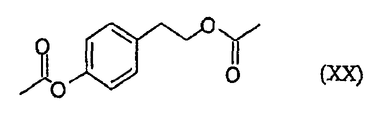

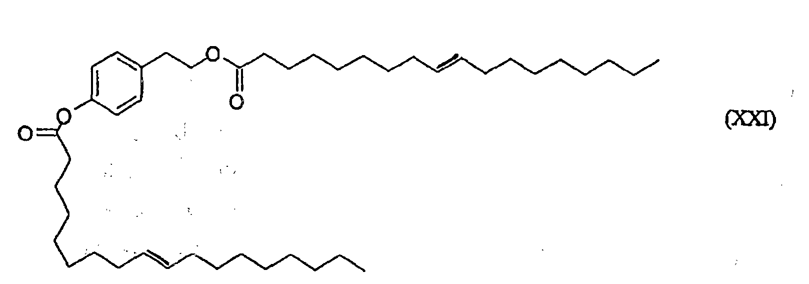

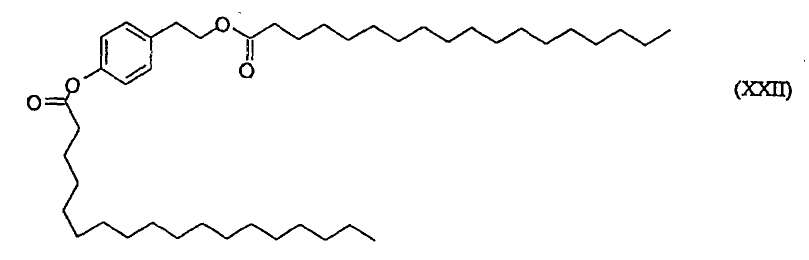

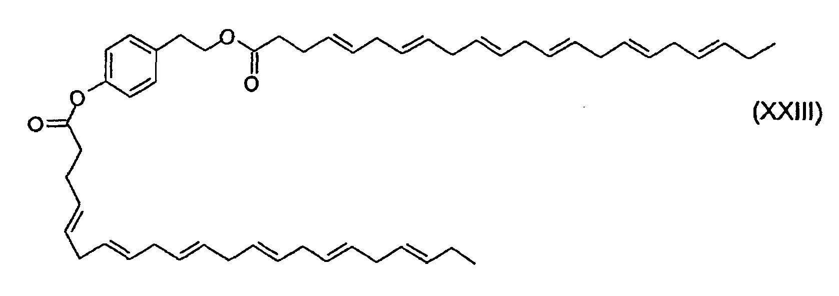

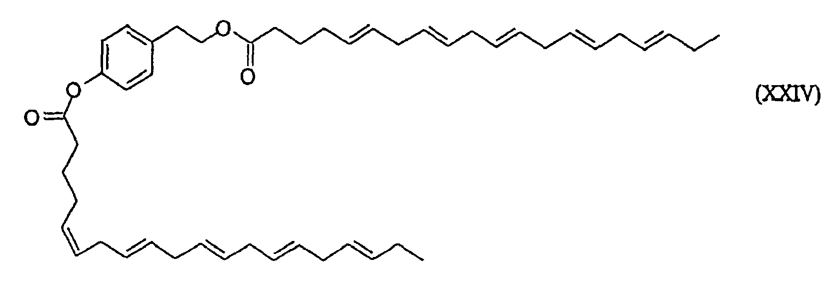

Hydroxytyrosol (reference compound) is represented by formula II and tyrosol (reference compound) is represented by formula III.

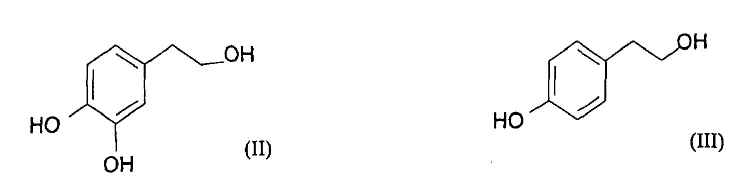

Hydroxytyrosol and tyrosol are sensitive to oxidation and are hydrophilic. This last characteristic might be problematic if hydroxytyrosol or tyrosol are pretended to be used in fat-based food products. Oxidation of hydroxytyrosol and tyrosol clearly affect the stability and preservation of both compounds. The hydroxytyrosol and tyrosol derivatives presented in this invention avoid these two problems. The hydroxyl groups on these derivatives are protected from oxidation by preparing hydroxytyrosol or tyrosol fatty acid esters. When compared to hydroxytyrosol and tyrosol, the hydroxytyrosol and tyrosol fatty acid esters are much more resistant against oxidation. At the same time, depending on the length of the fatty acid chain of the fatty acid esters, their solubility in fat-based food products will be increased. Hydroxytyrosol or tyrosol esters with a wide range of solubilities can be prepared, from totally watersoluble hydroxytyrosol or tyrosol derivatives when acetic acid is used in the formation of the ester to totally oil- soluble hydroxytyrosol or tyrosol derivatives when oleic acid is used in the formation of the ester. The hydroxytyrosol and tyrosol derivatives are hydrolysed in the intestinal tract of rats to their two components, hydroxytyrosol or tyrosol and the fatty acid. Hydroxytyrosol or tyrosol are then rapidly absorbed, being detected in plasma and cerebrospinal fluid. This implies that, after their hydrolysis, mentioned fatty acid esters can act as antioxidants to prevent diseases with an oxidative stress origin.

Oleuropein (reference compound) (formula IV) is a natural product found in olive, and like the fatty acid esters mentioned above, liberates hydroxytyrosol after its hydrolysis. Therefore, oleuropein is protected from oxidation, and because it liberates hydroxytyrosol in the stomach of a consumer, it is also useful in the treatment and prevention of neurodegenerative disease.

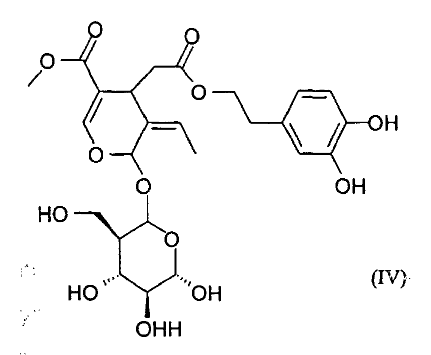

The second aspect of the invention relates to compositions comprising phenolic derivatives for the treatment of neurodegenerative diseases. Said compositions comprise esters of hydroxytyrosol, esters of tyrosol or combinations thereof, and may be pharmaceutical compositions or food compositions.

The pharmaceutical compositions of the invention may contain one or more appropriate binders, carriers and/or further auxiliary materials. The carrier materials, binders and/or auxiliary materials must be pharmaceutically and pharmacologically tolerable, so that they can be combined with the other components of the formulation or preparation and do not exert adverse effects on the organism treated.

The formulations include those, which are suitable for oral or parentheral (including subcutaneous, intradermal, intramuscular and intravenous) administration, even though the best route of administration is dependent on the patient's status.

The formulations can be in the form of single doses. The formulations are prepared according to methods known in the field of pharmacology. The appropriate quantities of active substances suitable for administration may vary as a function of the particularly field therapy. In general, the active substance concentration in a single-dose formulation is 5% to 95% of the total formulation.

The food compositions of the invention include any food composition to which compounds described in this invention have been added, or any food composition, which has been prepared in the presence of the compounds of the invention. Said compositions are preferably: milk shake, flavoured milk, milk, yoghurt, fermented milk, biscuit, juice, cake, bread, infant food, dehydrated food, oil, chewing gum, candies, or clinical nutrition formula.

The compounds of the invention can therefore be employed as nutritional supplements.

The invention provided by the application is illustrated by the examples herein below.

### EXAMPLES

### Example 1

### Cerebrospinal fluid

Several rats were given 100 mg hydroxytyrosol (reference compound) by oral administration. After 15 min a small amount of cerebrospinal fluid was taken from the rat and analysed for the presence of hydroxytyrosol. Also the total antioxidant capacity in this fluid was measured and compared with a control. The detection of hydroxytyrosol was accomplished by gas chromatography and concentrations were determined by a standard curve. Hydroxytyrosol reached a concentration between 0,1 and 0,2 mM in cerebrospinal fluid after 15 minutes of oral administration. Moreover, the total antioxidant capacity of cerebrospinal fluid increased by 35% under these conditions as compared to a control.

### Example 2

### Neuroprotection

The capacity of two phenolic compounds to rescue neuronal cells from different kind of stressors was tested. To primary hippocampal cultures from rat, different neurotoxins (glutamate, hydrogen peroxide or beta-amyloid peptide) were added in such concentrations that in half of the cells apoptosis was induced. Then, together with the toxins, hydroxytyrosol (reference compound) or tyrosol (reference compound) were added in a final concentration of 50 µM, and the cells were incubated for 24 h. at 37°C. Subsequently, apoptosis was measured in the cell cultures and the number of cells that were protected by this treatment as compared to a control was calculated. Both phenolic compounds tested protected part of the cells from dying, although hydroxytyrosol protected more cells than tyrosol did. Hydroxytyrosol protected around 60% of the cells against hydrogen peroxide, 35% against glutamate-induced neurotoxicity, and 45% against beta-amyloid induced apoptosis.

### Example 3

### Prevention of beta-amyloid precipitation

We found that hydroxytyrosol (reference compound) is able to bind iron, zinc and copper ions with high affinity. Therefore, an experiment was performed to study its capacity to solubilize beta-amyloid peptide from insoluble aggregates. Beta-amyloid peptide (1-40) was brought to 10 µM in 150 mM NaCl, 50 mM HEPES (pH 7,4), and mixed with 10 µM CuCl₂ or 10 µM FeCl₃. Precipitation of beta-amyloid peptide by copper and iron was measured by a turbidometric assay (Huang et al. J. Biol. Chem. 1997, 272(42), 26464-70). Copper- and iron-induced turbidity was almost completely abolished (>90%) by the presence of 25 µM hydroxytyrosol.

### Example 4

### Monoamine oxidase inhibition by hydroxytyrosol (reference compound)

We determined the IC50 of hydroxytyrosol for monoamine oxidase B (MAO-B). Therefore, MAO-B enzyme activities were measured using purified mitochondria from rat brain tissue as described previously (Holt et al. Anal. Biochem. 1997, 244, 384-392). Benzylamine (300 µM) was used as a substrate for MAO-B and the IC50 was calculated by adding different amounts of hydroxytyrosol to the enzyme assay. It was found that hydroxytyrosol is a strong inhibitor of MAO-B with an IC50 of 16.3 ± 1.3 µM.

### Example 5

### Absorption of hydroxytyrosol (reference compound) and derivatives

The absorption of hydroxytyrosol (II), and the ability of 2-(3,4-dihydroxyphenyl) ethyl acetate (V) and 2-(3,4-distearyloxyphenyl) ethyl stearate (XXII) to liberate hydroxytyrosol were studied in mice. Equivalent amounts of each compound (10 mg hydroxytyrosol, 13 mg 2-(3,4-dihydroxyphenyl) ethyl acetate and 70 mg 2-(3,4-distearyloxyphenyl) ethyl stearate) were given to mice by oral administration, and after 15 and 60 min. blood was collected from each animal. Plasma from each blood sample was subsequently extracted with ethyl acetate, and subjected to analysis by gas chromatography in combination with mass spectrometry. Both at 15 and 60 min after 2-(3,4-dihydroxyphenyl) ethyl acetate administration, the amount of hydroxytyrosol was very similar in plasma when compared to hydroxytyrosol administration (see table below).

| **compound administered** | **hydroxytyrosol in plasma, 15 min.** | **Hydroxytyrosol in plasma, 60 min.** |
|---|---|---|
| hydroxytyrosol (reference compound) | 0.79 mg/L | 0.05 mg/L |
| 2-(3,4-dihydroxyphenyl) ethyl acetate | 0.68 mg/L | 0.06 mg/L |
| 2-(3,4-distearyloxyphenyl) ethyl stearate | not detected | 0.44 mg/L |

When 2-(3,4-distearyloxyphenyl) ethyl stearate was given to mice, hydroxytyrosol was only detected in plasma one hour after administration. After 15 min. no hydroxytyrosol could be detected in plasma samples (see table above). These results indicate that, although at different rates, both 2-(3,4-dihydroxyphenyl) ethyl acetate and 2-(3,4-distearyloxyphenyl) ethyl stearate liberate hydroxytyrosol in the stomach or intestine of mice which can subsequently be taken up an pass to the blood stream.

### Example 6

### Preparation of an enriched juice

An enriched juice was prepared using the following ingredients:

| Ingredient | Amount per litre | |
|---|---|---|
| Concentrated juice | 200 | g |
| Tyrosol (reference compound) | 1 | g |
| Insoluble fibre | 10 | g |
| Lecithin | 0.5 | g |
| Flavours | 2 | g |
| Vitamin C | 90 | mg |
| Vitamin B1 | 2.1 | mg |
| Vitamin B2 | 2.4 | mg |
| Vitamin B6 | 3 | mg |
| Vitamin B12 | 1.5 | µg |
| Vitamin A | 1.2 | mg |
| Vitamin D | 7.5 | µg |
| Folic acid | 300 | µg |

### Processing technology

The final product was prepared from a concentrated juice by addition of water and water soluble ingredients. Then, tyrosol was added and mixed and the resulting product was pasteurised and homogenized. Finally, the product was cooled and packaged.

### Example 7

### Preparation of an U.H.T milk based product

A milk based product was prepared using the following ingredients:

| Ingredient | Amount per litre | |
|---|---|---|
| Skimmed milk | 960 | g |
| Skimmed milk powder | 17 | g |
| Hydroxytyrosol (reference compound) | 1 | g |
| Bisodium phosphate | 0.5 | g |
| Tripotasium phosphate | 0.2 | g |
| Water | 2 | g |
| Vitamin B6 | 3 | mg |
| Vitamin B12 | 3.8 | µg |
| Vitamin A | 1200 | µg |
| Vitamin D | 7.5 | µg |
| Vitamin E | 15 | mg |
| Folic acid | 300 | µg |

### Processing technology

All solid ingredients were mixed with the liquid milk and water. Then, hydroxytyrosol was admixed and homogenised in the absence of oxygen. The resulting dairy product was then subjected to U.H.T. treatment (150° C for 4 to 6 seconds) and finally packaged in the absence of oxygen.

### Example 8

### Preparation of a liquid nutritionally balanced diet formulation

A liquid nutritionally balanced diet formula was prepared using the following ingredients:

| Ingredient | Amount per litre | g |
|---|---|---|
| Skimmed milk | 814.3 | g |
| Whey protein concentrate | 11.8 | g |
| Oil blend | 32.5 | g |
| Sacarose | 27 | g |
| Maltodextrine | 71 | g |
| Soluble Fibre | 10 | g |
| Vitamin A | 1200 | mg |
| Vitamin D | 7.5 | µg |
| Vitamin E | 15 | mg |
| Vitamin K | 120 | µg |
| Vitamin C | 90 | mg |
| Thiamine | 2.25 | mg |
| Riboflavin | 2.55 | mg |
| Pyridoxine | 3 | mg |
| Vitamin B12 | 3 | µg |
| Niacin | 28.5 | mg |
| Folic acid | 30 | µg |
| Panthotenic acid | 4 | mg |
| Biotin | 40 | µg |
| Calcium | 1200 | mg |
| Phosphorus | 970 | mg |
| Magnesium | 47 | mg |
| Sodium | 480 | mg |
| Potassium | 1000 | mg |
| Chloride | 700 | mg |
| Iron | 43 | mg |
| Zinc | 24.5 | mg |
| Iodine | 150 | µg |
| Manganese | 100 | µg |
| Selenium | 10 | µg |
| Mono- and diglycerides | 1.5 | g |
| Bisodium phosphate (Na₂HPO₄ 5H₂O) | 0.6 | g |
| Carrageenan | 4 | g |
| Demineralised Water | 70 | g |
| Flavour | 2.75 | g |
| 2-(3,4-dihydroxyphenyl)ethyldocosahexaenoate | 1.5 | g |

### Processing technology

To an appropriately sized blend tank with agitation and heating all solid ingredients were mixed with the liquid milk and water. Then, 2-(3,4-dihydroxyphenyl) ethyl docosahexaenoate was admixed. The mixture was then heated at 60-70° C and emulsified through a single stage homogenizer at 6 to 7 MPa in absence of oxygen. After emulsification the mixture was heated to 140-150° C, 4-6 s, and was then passed through a two stages homogenizer (27-29 MPa and 3-4 MPa). Finally the mixture was packaged in absence of oxygen.

### Example 9

### Synthesis of 2-(3,4-dihydroxyphenyl) ethyl acetate (V)

Powdered anhydrous K₂CO₃ (90mg, 0.65 mmol), acetyl chloride (0.46 ml, 0.66 mmol) and tetrabutylammonium hydrogensulfate (TBAH, 22 mg, 0.06 mmol) were added to a stirred solution of 2-(3,4-dihydroxyphenyl) ethanol (100 mg, 0.65 mmol) in dry THE (5 ml). The mixture was stirred under argon at room temperature for 15 h. Then, the reaction mixture was filtered and evaporated to dryness. The residue was dissolved in dichloromethane (50 ml), washed with water (2x50 ml) and the organic layer was dried over anhydrous sodium sulphate and evaporated to dryness. The residue was purified by column chromatography using a solvent mixture of n-hexane: diethyl ether (1:1) to give 72 mg of a clear syrup in 57% yield.

¹H-NMR (300 mHz, CDCl₃): 6.78 (d, J=8.1 Hz, 1H, aromatic), 6.73 (d, J=1.5 Hz, 1H, aromatic), 6.63 (dd, J=8.0, 1.5 Hz, 1H, aromatic), 4.23 (t, J=7.1 Hz, 2H, -CH₂OOC-), 2.81 (t, J=7.1 Hz, 2H, ar-CH₂-), 2.03 (s, 3H, -CH₃).

### Example 10

### Synthesis of 2-(3,4-dihydroxyphenyl) ethyl stearate (VII)

Powdered anhydrous K₂CO₃ (90 mg, 0.65 mmol), stearoyl chloride (197 mg, 0.66 mmol) and 22 mg tetrabutylammonium hydrogensulfate (TBAH) were added to a stirred solution of 2-(3,4-dihydroxyphenyl) ethanol (100 mg, 0.65 mmol) in dry THF (5 ml). The mixture was stirred under argon at room temperature for 24 h. Then, the reaction mixture was filtrated and evaporated to dryness, solved in dichloromethane (50 ml) and washed with water (2x50 ml). The organic layer was dried over anhydrous sodium sulphate and evaporated to dryness. The residue was purified by column chromatography using a solvent mixture of n-hexane: diethyl ether (2:1) to give 132 mg of a white solid in 48% yield.

¹H-NMR (300 mHz, CDCl₃): 6.78 (d, J=8.1 Hz, 1H, aromatic), 6.72 (d, J=2, 1H, aromatic), 6.63 (dd, J=8.0, 2.0 Hz, 1H, aromatic), 5.34 (m, 2H, HC=CH), 4.22 (t, J=7.1 Hz, 2H, -CH₂OOC-), 2.81 (t, J=7.1 Hz, 2H, ar-CH₂-), 2.27 (t, J=7.4 Hz, 2H, -OOC-CH₂-), 1.58 (m, 2H, -OOC-CH₂- CH₂-), 1.24 (m, 28H, -CH₂-), 0.87 (t, J=6.9, 3H, -CH₃).

### Example 11

### Synthesis of 2-(3,4-dihydroxyphenyl)ethyl oleate (VI)

Powdered anhydrous K₂CO₃ (90mg, 0.65 mmol), cleoyl chloride (0.27 ml, 0.75 mmol) and tetrabutylammonium hydrogensulfate (TBAH) were added to a stirred solution of 2-(3,4-dihydroxyphenyl) ethanol (100 mg, 0.65 mmol) in dry THF (5 ml). The mixture was stirred under argon at room temperature for 24 h. Then, the reaction mixture was filtrated and evaporated to dryness, dissolved in dichloromethane (50 ml) and washed with water (2x50 ml). The organic layer was dried over anhydrous sodium sulphate and evaporated to dryness. The residue was purified by column chromatography using a solvent mixture of n-hexane: diethyl ether (4:1) to give 128 mg of lightly yellow syrup in 47% yield.

¹H-NMR (300 mHz, CDCl₃): 6.78 (d, J=8.1 Hz, 1H, aromatic), 6.72 (d, J=2, 1H, aromatic), 6.63(dd, J=8.0, 2.0 Hz, 1H, aromatic), 5.34 (m, 2H, HC=CH), 4.23 (t, J=7.1 Hz, 2H, -CH₂OOC-), 2.80 (t, J=7.1 Hz, 2H, ar-CH₂-), 2.28 (t, J=7.6 Hz, 2H, -OOC-CH₂-), 1.99 (m, 4H, -CH₂-HCCH- CH₂-), 1.58 (m, 2H, -OOC-CH₂-CH₂-), 1.26 (m, 26H, -CH₂-), 0.87 (t, J=6.9, 3H, -CH₃).

### Example 12

### Synthesis of 2-(3,4-diacetoxyphenyl) ethyl acetate (XV)

To a solution of 2-(3,4-dihydroxyphenyl) ethanol (200mg, 1.3 mmol) in dry THF (10 ml), pyridine (0.5 ml), acetic anhydride (0.6ml), and 4-dimethylaminopyridine (30 mg) were added. The mixture was stirred under argon at room temperature for 7 h. Then, methanol (25 ml) was added and the mixture was co-evaporated with toluene (3x10ml) to dryness. The crude was purified by column chromatography using a solvent mixture of n-hexane: diethyl ether (1:1) to give 136 mg of syrup in 75% yield.

¹H-NMR (300 mHz, CDCl₃): 7.10 (dd, J=10.2, 1.9 Hz, 2H, aromatic), 7.04 (s, 1H, aromatic), 4.26 (t, J=6.9 Hz, 2H,- CH₂OOC-), 2.89 (t, J=6.9 Hz, 2H ar-CH₂-), 2.27 (s, 3H, -ar-OCOCH₃), 2.26 (s, 3H, -ar-OCOCH₃), 2.02 (s, 3H, CH₃).

### Example 13

### Synthesis of 2-(3,4-distearoylphenyl) ethyl stearate (XVII)

To a stirred solution of 2-(3,4-dihydroxyphenyl) ethanol (100 mg, 0.65 mmol) in dry THF (10 ml), stearic acid (563 mg, 1.98 mmol), dicyclohexylcarbodiimide (410 mg, 1.98 mmol), and 4-dimethylaminopyridine (25 mg, 0.19 mmol) were added at 0° C. The mixture was stirred under argon at room temperature for 24 h. Precipitated urea was then filtered off and the filtrate evaporated to dryness. The residue was dissolved in dichloromethane (25 ml) and washed twice with 0.5 N HCl (2x50 ml), with saturated NaHCO₃ solution, and brine (1x50 ml), and finally dried over anhydrous sodium sulphate and evaporated to dryness. The residue was purified by column chromatography using a solvent mixture of n-hexane: diethyl ether (6:1) to give 200 mg (32%) of a white solid.

¹H-NMR (300 mHz, CDCl₃): 7.08 (m, AB system, 2H aromatic), 7.02 (s, 1H, aromatic), 4.26 (t, J=7.0 Hz, -CH₂OOC-), 2.91 (t, J=7.0 Hz, 2H ar-CH₂-), 2.50 (t, J=7.5, Hz 4H ar-OOC-CH₂-), 2.26 (t, J=7.5 Hz, 2H, -OOC-CH₂-), 1.71 (m, 4H, -ar-OOC-CH₂- CH₂-),1.62 (m, 2H, -OOC-CH₂- CH₂-), 1.24 (m, 84H -CH₂-), 0.87 (t, J=6.9, 9H, -CH₃).

## Claims

1. Phenolic derivatives, **characterised in that** they liberate hydroxytyrosol or tyrosol after their hydrolysis, selected from the group consisting of: 2-(3,4-dihydroxyphenyl) ethyl stearate (VII), 2-(4-hydroxyphenyl) ethyl acetate (X), 2-(4-hydroxyphenyl) ethyl oleate (XI), 2-(4-hydroxyphenyl) ethyl docosahexaenoate (XIII), 2-(4-hydroxyphenyl) ethyl eicosapentaenoate (XIV), 2-(3,4-diacetoxyphenyl) ethyl acetate (XV), 2-(3,4-dioleyloxyphenyl) ethyl oleate (XVI), 2-(3,4-distearyloxyphenyl) ethyl stearate (XVII), 2-(3,4-didocosahexaenoyloxyphenyl) ethyl docosahexaenoate (XVIII), 2-(3,4-dieicosapentaenoyloxyphenyl) ethyl eicosapentaenoate (XIX), 2-(4-acetoxyphenyl) ethyl acetate (XX), 2-(4-oleyloxyphenyl) ethyl oleate (XXI), 2-(4-stearyloxyphenyl) ethyl stearate (XXII), 2-(4-docosahexaenoyloxyphenyl) ethyl docosahexaenoate (XXIII), and 2-(4-eicosapentaenoyloxyphenyl) ethyl eicosapentaenoate (XXIV), for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

2. Phenolic derivative, **characterised in that** it liberates hydroxytyrosol after its hydrolysis, which is 2-(3,4-dihydroxyphenyl) ethyl acetate (V), for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

3. Phenolic derivative, **characterised in that** it liberates hydroxytyrosol after its hydrolysis, which is 2-(3,4-dihydroxyphenyl) ethyl oleate (VI), for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

4. Phenolic derivative, **characterised in that** it liberates hydroxytyrosol after its hydrolysis, which is 2-(3,4-dihydroxyphenyl) ethyl docosahexaenoate (VIII), for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

5. Phenolic derivative, **characterised in that** it liberates hydroxytyrosol after its hydrolysis, which is 2-(3,4-dihydroxyphenyl) ethyl eicosapentaenoate (IX), for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

6. Composition comprising one or more compounds as listed in claims 1 to 5 for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

7. Composition according to claim 6 **characterised in that** the composition is a pharmaceutical preparation.

8. Composition according claim 6 **characterised in that** the composition is a food composition to which a phenolic derivative as defined in claims 1 to 5 has been added.

9. Composition according to claim 8 **characterised in that** the food composition is a milk shake, a flavoured milk, a milk, a yoghurt, a fermented milk, a biscuit, a juice, a cake, a bread, an infant food, a dehydrated food, an oil, a chewing gum, a candy, or a clinical nutrition formula.

10. Use of phenolic derivatives, selected from the group consisting of: 2-(3,4-dihydroxyphenyl) ethyl stearate (VII), 2-(4-hydroxyphenyl) ethyl acetate (X), 2-(4-hydroxyphenyl) ethyl oleate (XI), 2-(4-hydroxyphenyl) ethyl stearate (XII), 2-(4-hydroxyphenyl) ethyl docosahexaenoate (XIII), 2-(4-hydroxyphenyl) ethyl eicosapentaenoate (XIV), 2-(3,4-diacetoxyphenyl) ethyl acetate (XV), 2-(3,4-dioleyloxyphenyl) ethyl oleate (XVI), 2-(3,4-distearyloxyphenyl) ethyl stearate (XVII), 2-(3,4-didocosahexaenoyloxyphenyl) ethyl docosahexaenoate (XVIII), 2-(3,4-dieicosapentaenoyloxyphenyl) ethyl eicosapentaenoate (XIX), 2-(4-acetoxyphenyl) ethyl acetate (XX), 2-(4-oleyloxyphenyl) ethyl oleate (XXI), 2-(4-stearyloxyphenyl) ethyl stearate (XXII), 2-(4-docosahexaenoyloxyphenyl) ethyl docosahexaenoate (XXIII), and 2-(4-eicosapentaenoyloxyphenyl) ethyl eicosapentaenoate (XXIV), **characterised in that** they liberate hydroxytyrosol or tyrosol after their hydrolysis, in the preparation of a composition for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

11. Use of 2-(3,4-dihydroxyphenyl) ethyl acetate (V), **characterised in that** it liberates hydroxytyrosol after its hydrolysis, in the preparation of a composition for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

12. Use of 2-(3,4-dihydroxyphenyl) ethyl oleate (VI), **characterised in that** it liberates hydroxytyrosol after its hydrolysis, in the preparation of a composition for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury; stroke, multiple sclerosis, loss of memory, or multiple infarct dementia.

13. Use of 2-(3,4-dihydroxyphenyl) ethyl docosahexaenoate (VIII), **characterised in that** it liberates hydroxytyrosol after its hydrolysis, in the preparation of a composition for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

14. Use of 2-(3,4-dihydroxyphenyl) ethyl eicosapentaenoate (IX), **characterised in that** it liberates hydroxytyrosol after its hydrolysis, in the preparation of a composition for the treatment or prevention of neurodegenerative diseases, as for example: Creutzfeldt-Jakob disease, Alzheimer's disease, Huntington's disease, Lewy body disease, Parkinson's disease, Pick's disease, amyotrophic lateral sclerosis, neurofibromatosis, brain injury, stroke, multiple sclerosis, loss of memory, or multiple infarct dementia.

15. Use according to claims 10 to 14 **characterised in that** the composition is a pharmaceutical preparation.

16. Use according to claims 10 to 14 **characterised in that** the composition is a food composition to which a compound as defined in claims 10 to 14 has been added.

17. Use according to claim 16 **characterised in that** the food composition is a milk shake, a flavoured milk, a milk, a yoghurt, a fermented milk, a biscuit, a juice, a cake, a bread, an infant food, a dehydrated food, an oil, a chewing gum, a candy, or a clinical nutrition formula.

18. Use of the compounds as listed in claims 1 to 5 and/or 10 to 14 as nutritional supplements.

## Patentansprüche

1. Phenolderivate, **gekennzeichnet durch** die Freisetzung von Hydroxytyrosol nach der Hydrolyse, ausgewählt aus der Gruppe bestehend aus: 2-(3,4-dihydroxy-phenyl) ethylstearat(VII), 2-(4-hydroxy-phenyl) ethylacetat (X), 2-(4-hydroxy-phenyl) ethyloleat (XI), 2-(4-hydroxyphenyl) ethyldocosahexaenoat (XIII), 2-(4-hydroxy-phenyl) ethyleicosapentaenoat (XIV), 2-(3,4-diacetoxy-phenyl) ethylacetat (XV), 2-(3,4-dioleyloxy-phenyl) ethyloleat (XVI), 2-(3,4-distearyloxy-phenyl) ethylstearat (XVII), 2-(3,4-didocosahexaenoyloxy-phenyl) ethyldocosahexaenoat (XVIII), 2-(3,4-dieicosapentaenoyloxy-phenyl) ethyleicosapentaenoat (XIX), 2-(4-acetoxy-phenyl) ethylacetat (XX), 2-(4-oleyloxy-phenyl) ethyloleat (XXI), 2-(4-stearyloxy-phenyl) ethylstearat (XXII), 2-(4-docosahexaenoyloxy-phenyl) ethyldocosahexaenoat (XXIII) und 2-(4-cicosapentaenoyloxy-phenyl) ethyleicosapentaenoat (XXIV) zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz.

2. Phenolderivat, **gekennzeichnet durch** die Freisetzung von Hydroxytyrosol nach der Hydrolyse, wobei es sich um 2-(3,4-dihydroxyphenyl) ethylacetat (V) zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz handelt.

3. Phenolderivat, **gekennzeichnet durch** die Freisetzung von Hydroxytyrosol nach der Hydrolyse, wobei es sich um 2-(3,4-dihydroxy phenyl) ethyloleat (VI) zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz handelt.

4. Phenolderivat, **gekennzeichnet durch** die Freisetzung von Hydroxytyrosol nach der Hydrolyse, wobei es sich um 2-(3,4-dihydroxyphenyl) ethyldocosahexaenoat (VIII), zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz handelt.

5. Phenolderivat, **gekennzeichnet durch** die Freisetzung von Hydroxytyrosol nach der Hydrolyse, wobei es sich um 2-(3,4-dihydroxyphenyl) ethylcicosapentaenoat (IX) zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz handelt

6. Zusammensetzung, die aus einem oder mehreren Stoffen gemäß den Patentansprüchen 1 bis 5 besteht, zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz.

7. Zusammensetzung gemäß Patentanspruch 6, **dadurch gekennzeichnet, dass** es sich um ein pharmazeutisches Präparat handelt.

8. Zusammensetzung gemäß Patentanspruch 6, **dadurch gekennzeichnet, dass** es sich um ein Nahrungsmittelpräparat handelt, dem ein Phenolderivat gemäß den Patentansprüchen 1 bis 5 zugefügt wurde.

9. Zusammensetzung gemäß Patentanspruch 8, **dadurch gekennzeichnet, dass** das Nahrungsmittelpräparat sich um ein Milchshake, eine aromatisierte Milch, ein Milchgetränk, einen Joghurt, eine fermentierte Milch, einen Keks, einen Saft, eine Torte, ein Brot, eine Kindernahrung, ein gefriergetrocknetes Nahrungsmittel, ein Öl, ein Kaugummi, ein Bonbon oder um ein Präparat für klinische Nahrung handelt.

10. Verwendung von Phenolderivaten, ausgewählt aus der Gruppe bestehend aus: 2-(3,4-dihydroxy-phenyl) ethylstearat (VII), 2- (4-hydroxyphenyl) ethylacetat (X), 2-(4-hydroxy-phenyl) ethyloleat (XI), 2-(4-hydroxy-phenyl) ethylstearat (XII), 2-(4-hydroxy-phenyl) ethyldocosahexaenoat (XIII), 2-(4-hydroxy-phenyl) ethyleicosapentaenoat (XIV), 2-(3,4-diacetoxy-pheny2) ethylacetat (XV), 2-(3,4-dioleyloxy-phenyl) ethyloleat (XVI), 2-(3,4-distearyloxy-phenyl) ethyl-stearat (XVII), 2-(3,4-didocosahexaenoyloxy-phenyl) ethyldocosahexaenoat (XVIII), 2-(3,4-dieicosapentaenoyloxy-phenyl) ethyleicosapentaenoat (XIX), 2-(4-acetoxyphenyl) ethylacetat (XX), 2-(4-oleyloxy-phenyl) ethyloleat (XXI), 2-(4-stearyloxy-phenyl) ethylstearat (XXII), 2-(4-docosahexaenoyloxy-phenyl) ethyldocosahexaenoat (XXIII) und 2-(4-eicosapentaenoyloxy-phenyl) ethyleicosapentaenoat (XXIV), **gekennzeichnet durch** die Freisetzung von Hydroxytyrosol oder Tyrosol nach der Hydrolyse bei der Herstellung einer Zusammensetzung zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz.

11. Verwendung von 2-(3,4-dihydroxyphenyl)ethylacetat (V), **gekennzeichnet durch** die Freisetzung von Hydroxytyrosol nach der Hydrolyse bei der Herstellung einer Zusammensetzung zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz.

12. Verwendung von 2-(3,4-dihydroxy-phenyl) ethyloleat (VI), **gekennzeichnet durch** die Freisetzung von hydroxytyrosol nach der Hydrolyse bei der Herstellung einer Zusammensetzung zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz.

13. Verwendung von 2-(3,4-dihydroxy-phenyl) ethyldocosahexaenoat (VIII) **gekennzeichnet durch** die Freisetzung von Hydroxytyrosol nach der Hydrolyse bei der Herstellung einer Zusammensetzung für die Behandlung oder zur Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Lateralsklerose, Neurofibromatose, Gehirnverletzung, Schlaganfall, Multiple Sklerose, Gedächtnisverlust oder Multi-Infarkt-Demenz.

14. Verwendung von 2-(3,4-dihydroxy-phenyl) ethyleicosapentaenoat (IX), **gekennzeichnet durch** die Freisetzung von Hydroxytyrosol nach der Hydrolyse bei der Herstellung einer Zusammensetzung zur Behandlung oder Vorbeugung von neurodegenerativen Krankheiten, wie zum Beispiel: Creutzfeldt-Jakob-Krankheit, Alzheimer-Krankheit, Huntington-Krankheit, Lewy-Körper-Krankheit, Parkinson-Krankheit, Pick-Krankheit, Amyotrophische Latcralsklerose, Neurofibromatose, Gehirnverletzung, Schlaganfall, Multiple Sklerose, Gedächtnisverlust oder Multi-Infarkt-Demenz.

15. Verwendung gemäß den Patentansprüchen 10 bis 14, **dadurch gekennzeichnet, dass** die Zusammensetzung ein pharmazeutisches Präparat ist.

16. Verwendung gemäß den Patentansprüchen 10 bis 14, **dadurch gekennzeichnet, dass** die Zusammensetzung ein Nahrungsmittelpräparat ist, dem ein Stoff gemäß den Patentansprüchen 10 bis 14 zugefügt wurde.

17. Verwendung gemäß Patentanspruch 16, **dadurch gekennzeichnet, dass** das Nahrungsmittelpräparat sich um ein Milchshake, eine aromatisierte Milch, ein Milchgetränk, einen Joghurt, eine fermentierte Milch, einen Keks, einen Saft, eine Torte, ein Brot, eine Kindernahrung, ein gefriergetrocknetes Nahrungsmittel, ein Öl, ein Kaugummi, ein Bonbon oder um ein Präparat für klinische Nahrung handelt.

18. Verwendung der Zusammensetzungen gemäß den Patentansprüchen 1 bis 5 und/oder 10 bis 14 als Nahrungsergänzungen.

## Revendications

1. Dérivés phénoliques, **caractérisés en ce qu'**ils libèrent de l'hydroxytyrosol ou tyrosol après son hydrolyse, sélectionnés parmi le groupe qui consiste en: stéarate de 2-(3,4-dihydroxy-phényl) éthyle (VII), acétate de 2-(4-hydroxy-phényl) éthyle (X), oléate de 2-(4- hydroxy-phényl) éthyle (XI), docosahexaénoate de 2-(4- hydroxy-phényl) éthyle (XIII), eicosapentaénoate de 2-(4-hydroxy-phényl) éthyle (XIV), acétate de 2-(3,4-diacétoxy-phényl) éthyle (XV), oléate de 2-(3,4-dioleyloxy-phényl) éthyle (XVI), stéarate de 2-(3,4-diestéaryloxy-phényl) éthyle (XVII), docosahexaénoate de 2-(3,4-didocosahexaénoyloxy-phényl) éthyle (XVIII), eicosapentaénoate de 2-(3,4-dieicosapentaénoyloxy-phényl) éthyle (XIX), acétate de 2-(4-acétoxy-phényl) éthyle (XX), oléate de 2-(4-oleyloxy-phényl) éthyle (XXI), stéarate de 2-(4-estéaryloxy-phényl) éthyle (XXII), docosahexaénoate de 2-(4-docosahexaénoyloxy-phényl) éthyle (XXIII) et eicosapentaénoate de 2-(4-cicosapentaénoyloxy-phényl) éthyle (XXIV), pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

2. Dérivé phénolique, **caractérisé en ce qu'**il libère de l'hydroxytyrosol après son hydrolyse, qui est l'acétate de 2-(3,4-dihydroxy-phényl) éthyle (V), pour le traitement ou la prévention de maladies neuro-dégénératives, la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence pour infarctus.

3. Dérivé phénolique, **caractérisé en ce qu'**il libère de l' hydroxytyrosol après son hydrolyse, qui est l'oléate de 2-(3,4-dihydroxy-phényl) éthyle (VI), pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

4. Dérivé phénolique, **caractérisé en ce qu'**il libère de l'hydroxytyrosol après son hydrolyse, qui est du docosahexaénoate de 2-(3,4-dihydroxy-phényl) éthyle (VIII), pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

5. Dérivé phénolique, **caractérisé en ce qu'**il libère de l' hydroxytyrosol après son hydrolyse, qui est l'eicosapentaénoate de 2-(3,4-dihydroxy-phényl) éthyle (IX), pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

6. Composition qui comprend un ou plusieurs des composants selon les revendications de 1 à 5, pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

7. Composition selon la revendication 6, **caractérisée en ce que** la composition est une préparation pharmaceutique.

8. Composition selon la revendication 6, **caractérisée en ce que** la composition est une composition alimentaire à laquelle il a été ajouté un dérivé phénolique selon les revendications de 1 à 5.

9. Composition selon la revendication 8, **caractérisée en ce que** la composition alimentaire est un lait battu, un lait aromatisé, un lait, un yaourt, un lait fermenté, un biscuit, un jus, une tarte, un pain, un aliment pour enfants, un aliment déshydraté, une huile, un chewing-gum, un bonbon ou une préparation pour nutrition clinique.

10. Utilisation de dérivés phénoliques, sélectionnés parmi le groupe qui consiste en: stéarate de 2-(3,4-dihydroxy-phényl) éthyle (VII), acétate de 2-(4-hydroxy-phényl) éthyle (X), oléate de 2-(4-hydroxy-phényl) éthyle (XI), stéarate de 2-(4-hydroxy-phényl) éthyle (XII), docosahexaénoate de 2-(4-hydroxy-phényl) éthyle (XIII), eicosapentaénoate de 2-(4-hydroxy-phényl) éthyle (XIV), acétate de 2-(3,4-diacétoxy-phényl) éthyle (XV), oléate de 2-(3,4-dioleyloxy-phényl) éthyle (XVI), stéarate de 2-(3,4-diestéaryloxy-phényl) éthyle (XVII), docosahexaénoate de 2-(3,4-didocosahexaénoyloxy-phényl) éthyle (XVIII), eicosapentaénoate de 2-(3,4-dieicosapentaénoyloxy-phényl) éthyle (XIX), acétate de 2-(4-acétoxy-phényl) éthyle (XX), oléate de 2-(4-oleyloxy-phényl) éthyle (XXI), stéarate de 2-(4-estéaryloxy-phényl) éthyle (XXII), docosahexaénoate de 2-(4-docosahexaénoyloxy-phényl) éthyle (XXIII) et eicosapentaénoate de 2-(4-eicosapentaénoyloxy-phényl) éthyle (XXIV), **caractérisée en ce qu'**ils libèrent de l'hydroxytycosol ou du tyrosol après leur hydrolyse, dans la préparation d'une composition pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

11. Utilisation de l'acétate de 2-(3,4-dihydroxy-phényl) éthyle (V), **caractérisé en ce qu'**il libère de l'hydroxytyrosol après son hydrolyse, dans la préparation d'une composition pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

12. Utilisation de l'oléate de 2-(3,4-dihydroxy-phényl) éthyle (VI), **caractérisé en ce qu'**il libère de l'hydroxytyrosol après son hydrolyse, dans la préparation d'une composition pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

13. Utilisation du docosahexaénoate de 2-(3,4-dihydroxy-phényl) éthyle (VIII), **caractérisé en ce qu'**il libère de l'hydroxytyrosol après son hydrolyse, dans la préparation d'une composition pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

14. Utilisation de l'cicosapentaénoate de 2-(3,4-dihydroxy-phényl) éthyle (IX), **caractérisé en ce qu'**il libère de l'hydroxytyrosol après son hydrolyse, dans la préparation d'une composition pour le traitement ou la prévention de maladies neuro-dégénératives, comme par exemple: la maladie de Creutzfeldt-Jakob, la maladie d'Alzheimer, la maladie de Huntington, la maladie avec des corps de Lewy, la maladie de Parkinson, la maladie de Pick, la sclérose latérale amyotrophique, neurofibromatose, lésion cérébrale, accident cérébro-vasculaire, sclérose multiple, perte de mémoire ou démence due à des infarctus multiples.

15. Utilisation selon les revendications de 10 à 14, **caractérisée en ce que** la préparation est une préparation pharmaceutique.

16. Utilisation selon les revendications de 10 à 14, **caractérisée en ce que** la composition est une composition alimentaire à laquelle il a été ajouté un composé selon les revendications de 10 à 14.

17. Utilisation selon la revendication 16, **caractérisée en ce que** la composition alimentaire est un lait battu, un lait aromatisé, un lait, un yaourt, un lait fermenté, un biscuit, un jus, une tarte, un pain, un aliment pour enfants, un aliment déshydraté, une huile, un chewing-gum, un bonbon ou une préparation pour nutrition clinique.

18. Utilisation des composés selon les revendications de 1 à 5 et/ou de 10 à 14, comme compléments nutritionnels.
